# EUROPEAN PATENT APPLICATION

(11) **EP 2 572 742 A1**
(43) Date of publication of application: **27.03.2013**
(21) Application number: 11182623.6
(22) Date of filing: 23.09.2011
(51) Int. Cl.: A61M 5/32

(54) **Needle safety device**

(71) Applicant: Sanofi-Aventis Deutschland GmbH, 65929 Frankfurt (DE)
(72) Inventor: The designation of the inventor has not yet been filed

(57) **Abstract**

Described is a needle safety device (3), comprising a support body (3.1) having a first aperture (3.3) adapted to allow passage of a needle (2), and a needle shield (3.2) having a cover face (3.16) and a distal face (3.5) and a second aperture (3.4) formed in the distal face (3.5) adapted to allow passage of the needle (2). The needle shield (3.2) is hingedly mounted on the support body (3.1) and movable between a first angular position (P1) relative to the support body (3.1) in which the cover face (3.16) covers the first aperture (3.3) and a second angular position (P2) relative to the support body (3.1) in which the first aperture is aligned with the second aperture (3.4). When the needle shield (3.2) is in the second angular position (P2), the needle shield (3.2) is movable between a first axial position (PA1) relative to the support body (3.1) in which the needle (2) is within the support body (3.3) and a second axial position (PA2) relative to the support body (3.1) in which the needle (2) passes through the first aperture (3.3) and the second aperture (3.4).

## Description

### Background of the Invention

Medicament delivery devices (e.g., pen injectors, syringes, auto-injectors, etc.) that contain a selected dosage of a medicament are well known devices for administering the medicament to a patient. Safety devices for covering a needle of the delivery device before and after use are also well known. Typically, a needle shield of the safety device is either manually moved or automatically to surround the medical needle. Various attempts have been made to develop an optimally sized and functioning safety device. However, there remains a need for an optimal safety needle assembly.

### Summary of the Invention

It is an object of the present invention to provide an improved safety needle assembly that minimizes the risk of an accidental needle stick injury, that is safe to handle, and that provides needle safety before and after the medicament is delivered.

In an exemplary embodiment, a needle safety device comprises a support body having a first aperture adapted to allow passage of a needle, and a needle shield having a cover face and a distal face and a second aperture formed in the distal face adapted to allow passage of the needle. The needle shield is hingedly mounted on the support body and movable between a first angular position relative to the support body in which the cover face covers the first aperture and a second angular position relative to the support body in which the first aperture is aligned with the second aperture. When the needle shield is in the second angular position, the needle shield is movable between a first axial position relative to the support body in which the needle is within the support body and a second axial position relative to the support body in which the needle passes through the first aperture and the second aperture.

In an exemplary embodiment, the needle shield includes a bearing element formed adjacent the cover face.

In an exemplary embodiment, the needle safety device further comprises a first spring element in the support body which deflects when the needle shield is moved from the first axial position into the second axial position. The first spring element causes movement of the needle shield from the second angular position to the first angular position when the needle shield is moved from the second axial position to the first axial position. The needle shield includes arms to pass through slots formed in a distal end of the support body, and the arms deflect the first spring element when the needle shield is moved from the first axial position into the second axial position.

In an exemplary embodiment, the needle safety device further comprises a second spring element in the support body which compresses when the needle shield is moved from the first axial position into the second axial position. The second spring element abuts an inner bearing formed on the support body and a distal end of a needle hub or an injection device.

In an exemplary embodiment, guide tracks are formed in the support body and guide pins are formed on the needle shield, wherein the guide pins are adapted to engage the guide tracks. The guide pins are adapted to move axially in the guide tracks when the needle shield is moved from the first axial position into the second axial position. The needle shield is in second axial position, the guide pins are in indent portions (3.14) of the guide tracks, wherein the guide pins are adapted to rotate in the indent portions.

In an exemplary embodiment, when the needle shield is moved from the second axial position into the first axial position, the first spring element applies force to the arms causing the guide pins to rotate in the indent portions and thereby rotating the needle shield into the first angular position. The guide tracks include resilient locking elements which prevent rotation of the guide pins relative to the guide tracks when the guide pins have attained a predetermined angular position relative to the guide tracks. Rotation of the guide pins displaces the locking elements until the guide pins reach the predetermined angular position. When the guide pins reach the predetermined angular position, the locking elements deflect into the indent portions to form an abutment to prevent rotation of the guide pins in an opposite direction.

In an exemplary embodiment, the support body is adapted to engage a needle hub or an injection device.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an exemplary embodiment of a needle safety device in an exploded view;
- Figure 2: shows an exemplary embodiment of a needle safety device in an isometric view before use;
- Figure 3: shows an exemplary embodiment of a needle safety device in an isometric view after use;
- Figure 4: shows a sectional view of an exemplary embodiment of a needle safety device before use;
- Figure 5: illustrates the interaction of a guide pin with a guide track corresponding to the arrangement of an exemplary embodiment of a needle shield and a support body as shown in figure 4;
- Figure 6: shows a sectional view of an exemplary embodiment of a needle safety device during a drug delivery stage;
- Figure 7: illustrates the interaction of the guide pin with the guide track corresponding to the arrangement of the needle shield and the support body as shown in figure 6;
- Figure 8: shows a sectional view of an exemplary embodiment of a needle safety device during after use;
- Figure 9: illustrates the interaction of an exemplary embodiment of the guide pin with the guide track corresponding to the arrangement of the needle shield and the support body as shown in figure 8;
Corresponding parts are marked with the same reference symbols in all figures.

### Detailed Description

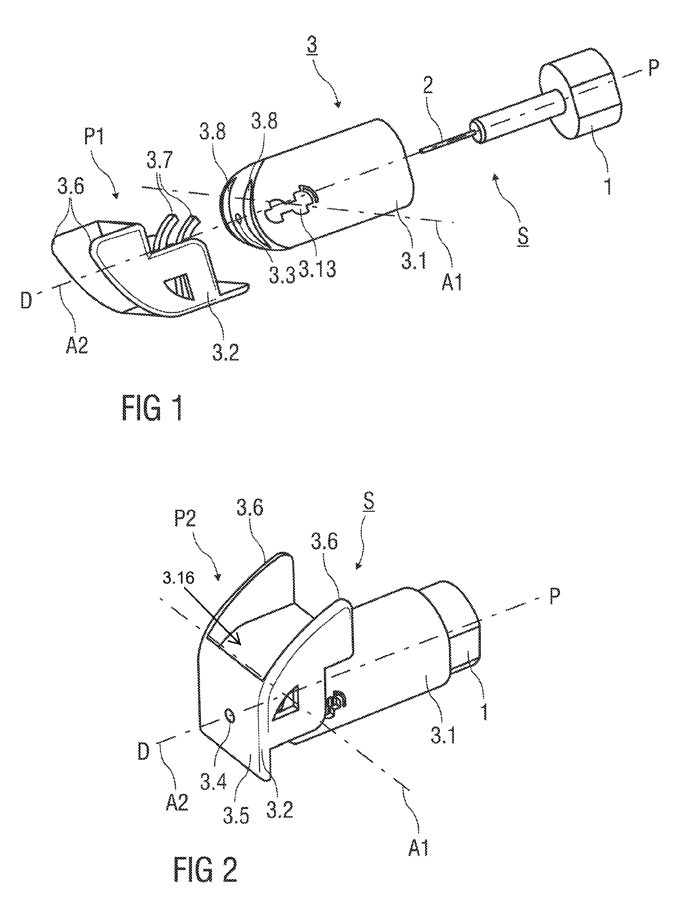

Figures 1 to 9 illustrate exemplary embodiments of a safety needle assembly S according to the present invention. In an exemplary embodiment, the needle assembly S comprises a needle hub 1 connected to a needle 2 and a needle safety device 3. The safety device 3 comprises a support body 3.1 that is adapted to be mounted to, or integrally formed with, the needle hub 1. A needle shield 3.2 is hingedly coupled to the support body 3.1 so as to allow for a pivoting movement of the needle shield 3.2 with respect to the support body 3.1.

In an alternative embodiment, the medical needle 2 may be attached to distal end of a medicament delivery device, such as for example, a pre-filled syringe, a pen injector, an auto-injector, etc. The support body 3.1 may engage the delivery device.

The safety device 3 is adapted to provide needle safety before and after delivery of the medicament. The safety device 3 is adapted to reduce the risk of accidental needle stick injuries before, during and after the medicament is delivered to the patient. Figures 1 shows an exemplary embodiment of the needle assembly S according to the present invention. In an exemplary embodiment, the safety device 3 is removably mounted to the needle hub 1 or an injection device. In another exemplary embodiment, the safety device 3 may be integrally formed with the needle hub 1 or the injection device.

In an exemplary embodiment, the safety device 3 includes a needle shield 3.2 which is hingedly mounted on a support body 3.1. The support body 3.1 includes a proximal end which is adapted to receive the needle hub 1 and/or the injection device. In an exemplary embodiment, the proximal end of the support body 3.1 may be coupled to the needle hub 1 by a snap fit, a threaded fit, a bayonet fit, a friction fit, etc. Prior to use the needle 2 may be retracted within the support body 3.1 and thus, hidden from view of the patient. This may help to overcome a patient's needle anxiety or phobia and thus may reduce the risk that the medical intervention is improperly carried out, in particular when the medicament is self-administered.

A distal end of the support body 3.1 may include a first aperture 3.3 which is adapted to allow the needle 2 to passthrough when the needle 2 is in an extended position relative to the support body 3.1.

One or more guide tracks 3.13 may be formed in a sidewall of the support body 3.1. In an exemplary embodiment, two guide tracks 3.13 may be formed in the sidewall of the support body 3.1 at 180 degrees spacing from each other. As explained further below, the guide tracks 3.13 may be used to provide a hinged connection for the needle shield 3.2 to the support body 3.1. A first axis A1 may be defined as extending crosswise across the support body 3.1 through the guide tracks 3.13. One or more slots 3.8 may be formed in the distal end of the support body 3.1. The slots 3.8 may be adapted to receive portions of the needle shield 3.2 to allow the needle shield 3.2 to rotate relative to the support body 3.1.

In an exemplary embodiment, the needle shield 3.2 includes one or more arms 3.7 extending proximally which are inserted in the slots 3.8. As shown in Figure 2, a distal end of the needle shield 3.2 may include a distal face 3.5 with a second aperture 3.4 formed therein. One or more bearing elements 3.6 may be formed adjacent to the distal face 3.5. A cover face 3.16 may be coupled to the distal face 3.5 at approximately 90 degrees, e.g., forming an L-shape.

Referring back to the exemplary embodiment shown in Figure 1, the needle shield 3.2 is shown positioned in a first angular position P1 relative to the support body 3.1. In the first angular position P1, the first aperture 3.3 of the support body 3.1 is covered by the cover face 3.16 of the needle shield 3.2 and thus penetration of the needle 2 into the patient is prevented. Figure 1 shows an exemplary embodiment of a pre-use position of the safety device 3. In this exemplary embodiment, the patient may be required to rotate the needle shield 3.2 relative to the support body 3.1 (e.g., by applying rotational force to the bearing element 3.6) to align the first and second apertures 3.3, 3.4 to prepare the needle assembly S for an injection. Inadvertent contact with the needle 2 may therefore be avoided. The safety device 3 may be packaged with the needle shield 3.2 in the first angular position P1.

Figure 2 shows a perspective view of an exemplary embodiment of the safety device 3 prior to injection. In this exemplary embodiment, the needle shield 3.2 is positioned in a second angular position P2 relative to the support body 3.1. In the second angular position P2, the needle shield 3.2 is rotated 90 degrees relative to the support body 3.1, and thus the second aperture 3.4 in the distal face 3.5 of the needle shield 3.2 is aligned with the first aperture 3.3 of the support body 3.1. In the second angular position, the distal face 3.5 extends in a plane normal to a second axis A2, which is parallel to a longitudinal axis of the safety device 3 and perpendicular to the first axis A1.

In an exemplary embodiment, the needle shield 3.2 may be manually rotated (e.g., by applying a rotational force to the bearing element 3.6) into the second angular position P2 to prepare the needle assembly S for an injection. In another exemplary embodiment, the safety device 3 may be packaged with the needle shield 3.2 in the second angular position P2. A peelable film (not shown) may be adhered to the distal face 3.5 to cover the second aperture 3.4 prior to use of the needle assembly S.

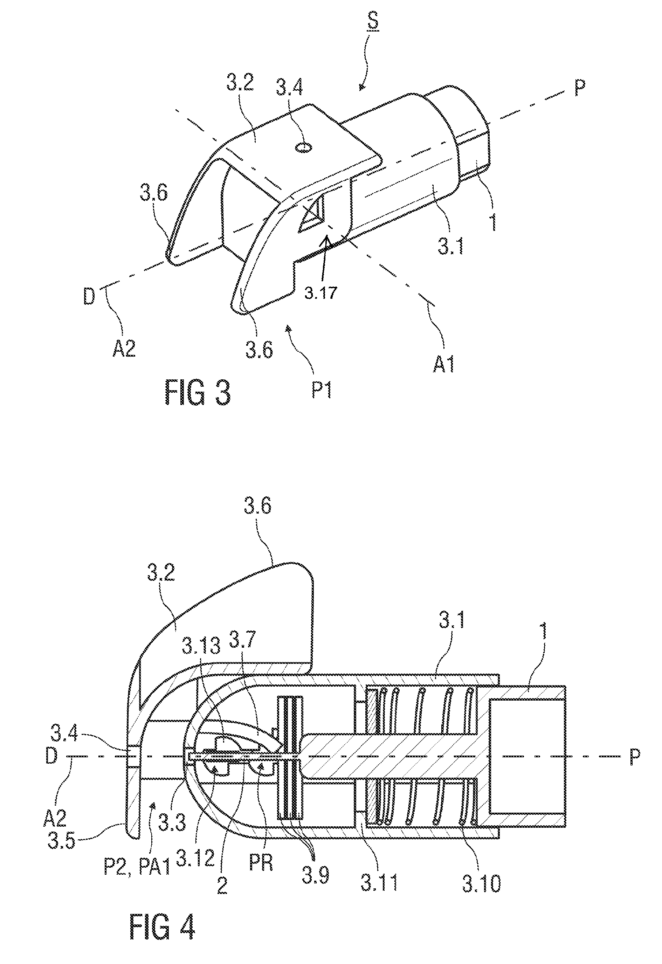

Figure 3 shows an isometric view of an exemplary embodiment of the safety device 3 in a post-use state after the injection has been performed. The needle 2 is housed within the support body 3.1, and the needle shield is permanently locked in the first angular position P1 covering the first aperture 3.3 of the support body 3.1 so as to block a re-exposure of the needle 2. Accidental needle stick injuries with used needles 2 may be efficiently avoided so that the risk of transmitting blood-borne diseases may be reduced.

Figure 4 shows an exemplary embodiment of the safety device 3 in a sectional view prior to injection, when the needle 2 is in a retracted position PR. Prior to use, the needle shield 3.2 is in the second angular position P2 such that the first and second apertures 3.3, 3.4 are aligned. The distal face 3.5 of the needle shield 3.2 is in a first axial position PA1 spaced away from the distal end of the support body 3.1. The arms 3.7 protrude through slots 3.8 into the support body 3.1, and proximal ends of the arms 3.7 abut against a first spring element 3.9 arranged within the support body 3.1. As will be explained further below, movement of the needle shield 3.2 in the proximal direction P relative to the support body 3.1 (e.g., during an injection) causes the arms 3.7 to deflect the first spring element 3.9. When pressure is released from the needle shield 3.2 (e.g., after the injection), the first spring element 3.9 applies a force to the arms 3.7 to rotate the needle shield 3.2 relative the support body 3.1. In the exemplary embodiment shown in figure 4, the spring element 3.9 are arranged as leaf springs and are made from a resilient plastic material. In another exemplary embodiment, the first spring element 3.9 may be a torsion spring.

A second spring element 3.10 is arranged within the support body 3.1. In the exemplary embodiment shown in Figure 4, the second spring element 3.10 is designed as a compression spring made from a metal. A distal end of the second spring element 3.10 abuts an inner bearing 3.11 formed on an inner surface of the support body 3.1. A proximal end of the second spring element 3.10 abuts a distal surface of the needle hub 1.

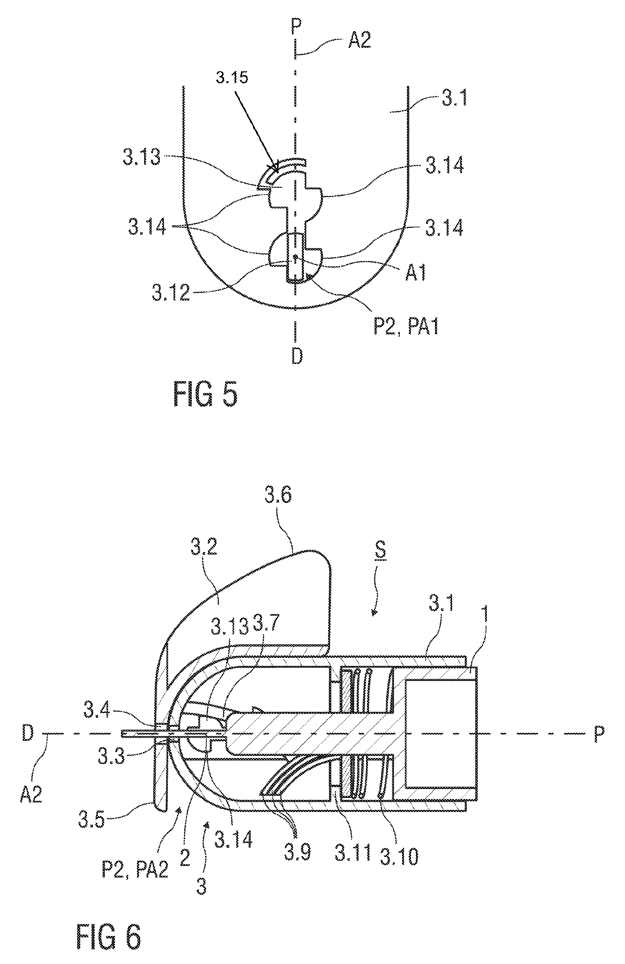

Figure 5 shows an exemplary embodiment of a hinge joint which allows the needle shield 3.2 to rotate relative to the support body 3.1. The hinge joint includes the guide track 3.13 formed in the side wall of the support body 3.1. A guide pin 3.12 formed on a leg 3.17 of the needle shield 3.2 engages the guide track 3.13. Those of skill in the art will understand that two guide pins 3.12 may engage corresponding guide tracks 3.13 formed in the support body 3.1. In the exemplary embodiment shown in Figure 5, the needle shield 3.2 is in the first axial position PA1 and the second angular position 2 relative to the support body 3.1, and the guide pin 3.12 abuts a distal portion of the guide track 3.13. The guide track 3.13 includes indent portions 3.14 (e.g., a distal indent portion and a proximal indent portion) which allow the guide pin 3.12 to rotate within the guide track 3.13. When the guide pin 3.12 is in the distal indent portion 3.14, the needle shield 3.2 is in the first axial position PA1, and when the guide pin 3.12 is in the proximal indent portion 3.14, the needle shield 3.2 is in the second axial position PA2. The guide track 3.13 prevents rotation of the guide pin 3.12 when the guide pin 3.12 is between the indent portions 3.14.

A locking element 3.15 arranged as a resilient detent adjacent the proximal indent portion 3.14 of the guide track 3.13. The locking element 3.15 is biased radially toward a center of the proximal indent portion 3.14 such that the guide pin 3.13 must apply a force to displace the locking element 3.15 if the guide pin 3.13 is going to rotate in the proximal indent portion 3.14.

Figure 6 shows an exemplary embodiment of the needle assembly S during an injection. Due to force applied to the distal face 3.5 (e.g., when the distal face 3.5 is applied to the injection site), the needle shield 3.2 is moved into the second axial position PA2 relative to the support body 3.1 by the arms 3.7 deflecting the first spring element 3.9. Further, the needle shield 3.2 abuts the distal end of the support body 3.1 which causes the second spring element 3.10 to compress, thereby exposing the needle 2 into the advanced position PA.

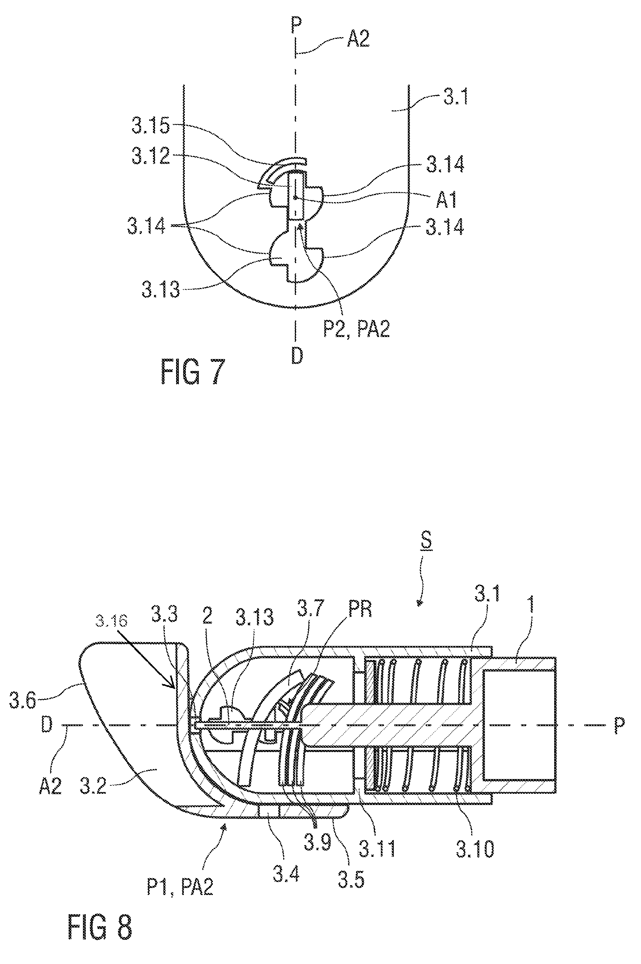

Figure 7 shows the location of the guide pin 3.12 within the guide track 3.13 when the needle shield 3.2 is in the second axial position PA2. The guide pin 3.12 has moved axially in a proximal direction into the proximal indent portion 3.14.

Figure 8 shows a sectional view of the safety needle assembly S in a needle safe state after the injection. As the force applied to the distal face 3.5 is removed, the second spring element 3.10 expands moving the support body 3.1 distally relative to the needle hub 1, thereby retracing the needle 2 into the support body 3.1 into the retracted position PR. As the needle 2 is retracted into the support body 3.1, the first spring element 3.9 returns to its undeflected state, which pushes the arms 3.7 causing the guide pin 3.12 to rotate in the proximal indent portion 3.14 in the guide track 3.13. Rotation of the guide pin 3.12 causes the needle shield 3.2 to rotate from the second angular position P2 into the first angular position P1. After the injection, when the needle shield is in the first angular position P1, the cover face 3.16 covers the first aperture 3.3 and prevents exposure of the needle 2.

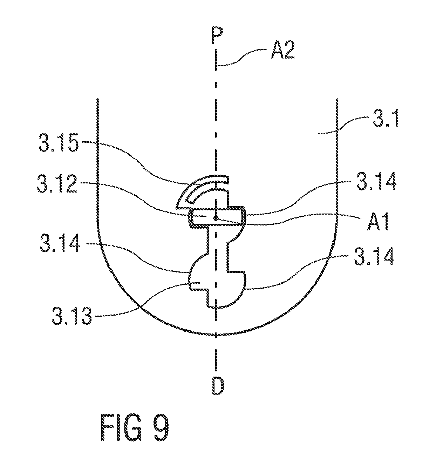

As shown in Figure 9, as the guide pin 3.12 rotates in the proximal indent portion 3.14, the locking element 3.15 is deflected until the guide pin 3.12 reaches a predetermined angular position relative to the guide track 3.13 (e.g., 90 degrees). When the guide pin 3.12 has reached the predetermined angular position (determined by an abutment face formed in the guide track 3.13), the locking element 3.15 is released and deflects radially toward a center of the proximal indent portion 3.14. The locking element 3.15 abuts the guide pin 3.12 and prevents rotation of the guide pin 3.12 in a reverse direction, and prevents a subsequent axial translation of the needle shield 3.2 with respect to the support body 3.1. The needle shield 3.2 is thus irreversibly locked in the second axial and first angular position PA2, P1 and the medical needle 2 is permanently retained in a needle safe state after the injection.

The safety needle assembly S with the safety device 3 is adapted to minimize the risk of accidental needle stick injuries. The medical needle 2 is hidden from the view of the patient throughout the medical intervention, which may help to alleviate a patient's fear of needles. The safety needle assembly S is simple to use. In particular, the user of the safety needle assembly S simply pushes the safety device 3 against the skin surface of the patient. A proper sequential movement of the various parts of the safety device 3 responsible for needle insertion, needle retraction and locking of the medical needle 2 in a needle safe state is automatically achieved by a purely mechanical interaction of the components of the safety needle assembly S.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle safety device (3), comprising:
a support body (3.1) having a first aperture (3.3) adapted to allow passage of a needle (2); and
a needle shield (3.2) having a cover face (3.16) and a distal face (3.5) and a second aperture (3.4) formed in the distal face (3.5) adapted to allow passage of the needle (2), the needle shield (3.2) hingedly mounted on the support body (3.1) and movable between a first angular position (P1) relative to the support body (3.1) in which the cover face (3.16) covers the first aperture (3.3) and a second angular position (P2) relative to the support body (3.1) in which the first aperture is aligned with the second aperture (3.4), and
wherein, when the needle shield (3.2) is in the second angular position (P2), the needle shield (3.2) is movable between a first axial position (PA1) relative to the support body (3.1) in which the needle (2) is within the support body (3.3) and a second axial position (PA2) relative to the support body (3.1) in which the needle (2) passes through the first aperture (3.3) and the second aperture (3.4).

2. The needle safety device (3) according to claim 1, wherein the needle shield (3.2) includes a bearing element (3.6) formed adjacent the cover face (3.16).

3. The needle safety device (3) according to any one of the preceding claims, further comprising:
a first spring element (3.9) in the support body (3.1) which deflects when the needle shield (3.2) is moved from the first axial position (PA1) into the second axial position (PA2).

4. The needle safety device (3) according to claim 3, wherein the first spring element (3.9) causes movement of the needle shield (3.2) from the second angular position (P2) to the first angular position (P1) when the needle shield (3.2) is moved from the second axial position (PA2) to the first axial position (PA1).

5. The needle safety device (3) according to claim 3, wherein the needle shield (3.2) includes arms (3.7) to pass through slots (3.8) formed in a distal end of the support body (3.1), and the arms (3.7) deflect the first spring element (3.9) when the needle shield (3.2) is moved from the first axial position (PA1) into the second axial position (PA2).

6. The needle safety device (3) according to any one of the preceding claims, further comprising:
a second spring element (3.10) in the support body (3.1) which compresses when the needle shield (3.2) is moved from the first axial position (PA1) into the second axial position (PA2).

7. The needle safety device (3) according to claim 5, wherein the second spring element (3.10) abuts an inner bearing formed on the support body (3.1) and a distal end of a needle hub (1) or an injection device.

8. The needle safety device (3) according to any one of the preceding claims, wherein guide tracks (3.13) are formed in the support body (3.1) and guide pins (3.12) are formed on the needle shield (3.2), wherein the guide pins (3.12) are adapted to engage the guide tracks (3.13).

9. The needle safety device (3) according to claim 8, wherein the guide pins (3.12) are adapted to move axially in the guide tracks (3.13) when the needle shield (3.2) is moved from the first axial position (PA1) into the second axial position (PA2).

10. The needle safety device (3) according to claim 9, wherein, when the needle shield is in second axial position (PA2), the guide pins (3.12) are in indent portions (3.14) of the guide tracks (3.13), wherein the guide pins (3.12) are adapted to rotate in the indent portions (3.14).

11. The needle safety device (3) according to claims 5 and 10, wherein, when the needle shield (3.2) is moved from the second axial position (PA2) into the first axial position, the first spring element (3.9) applies force to the arms (3.7) causing the guide pins (3.12) to rotate in the indent portions (3.14) and thereby rotating the needle shield into the first angular position (P1).

12. The needle safety device (3) according to any of claims 8 through 11, wherein the guide tracks (3.13) include resilient locking elements (3.15) which prevent rotation of the guide pins (3.12) relative to the guide tracks (3.13) when the guide pins (3.12) have attained a predetermined angular position relative to the guide tracks (3.13).

13. The needle safety device (3) according to claim 12, wherein rotation of the guide pins (3.12) displaces the locking elements (3.15) until the guide pins (3.12) reach the predetermined angular position.

14. The needle safety device (3) according to claim 13, wherein, when the guide pins (3.12) reach the predetermined angular position, the locking elements (3.15) deflect into the indent portions (3.14) to form an abutment to prevent rotation of the guide pins (3.12) in an opposite direction.
The needle safety device (3) according to any one of the preceding claims, wherein the support body (3.1) is adapted to engage a needle hub (1) or an injection device.
